(19) Europäisches Patentamt
European Patent Office
Office européen des brevets

(11) Numéro de publication : 0 478 471 B1

(12) **FASCICULE DE BREVET EUROPEEN**

(45) Date de publication du fascicule du brevet : **25.05.94 Bulletin 94/21**

(51) Int. Cl.$^5$ : **C07C 69/593,** C07C 67/37

(21) Numéro de dépôt : **91420325.2**

(22) Date de dépôt : **13.09.91**

# (54) Procédé de préparation de diesters de l'acide hexènedioique à partir de dialcoxy-1,2-butène-3.

(30) Priorité : **24.09.90 FR 9012042**

(43) Date de publication de la demande : **01.04.92 Bulletin 92/14**

(45) Mention de la délivrance du brevet : **25.05.94 Bulletin 94/21**

(84) Etats contractants désignés : **BE DE ES FR GB IT NL**

(56) Documents cités :
**EP-A- 0 217 407**
**EP-A- 0 347 340**

(73) Titulaire : **RHONE-POULENC CHIMIE**
**25, quai Paul Doumer**
**F-92408 Courbevoie Cédex (FR)**

(72) Inventeur : **Mutez, Sylvain**
**5, Hameau des Presles**
**F-69540 Irigny (FR)**
Inventeur : **Perron, Robert**
**La Pecolière**
**F-69390 Charly (FR)**
Inventeur : **Jenck, Jean**
**1, Impasse du Rotagnier**
**F-69680 Chassieu (FR)**
Inventeur : **Deweerdt, Hélène**
**89, rue Garibaldi**
**F-69006 Lyon (FR)**
Inventeur : **Kalck, Philippe**
**8, Allée "La Pradine" Auzeville-Tolozane**
**F-31320 Castanet Tolosane (FR)**

(74) Mandataire : **Vignally, Noel et al**
**Rhône-Poulenc Chimie**
**Direction de la Propriété Industrielle**
**Centre de Recherches des Carrières**
**B.P. 62**
**F-69192 Saint-Fons Cédex (FR)**

EP 0 478 471 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet un procédé de préparation de diesters de l'acide hexènedioïque-1,6. Ces diesters peuvent être hydrogénés en diesters de l'acide adipique correspondants ou adipates qui peuvent alors être hydrolysés pour former l'acide adipique. L'acide adipique, l'une des matières premières du nylon 66 est produit avec de forts tonnages et de ce seul fait, toute nouvelle voie d'accès à ce diacide et/ou à ses dérivés présente un intérêt de principe immédiatement perceptible.

La présente invention a plus spécifiquement pour objet un procédé de préparation de diesters de l'acide hexène-3-dioïque-1,6 par réaction du monoxyde de carbone sur au moins un dialcoxy-1,2-butène-3 en présence d'un catalyseur à base de palladium.

Il a déjà été proposé par IMAMURA et TSUJI, Tetrahedron vol. 25 p. 4187-4195 (1969) de préparer des diesters de l'acide hexène-3-dioïque-1,6 par réaction du monoxyde de carbone sur le diéthoxy-1,4-butène-2 dans l'éthanol, en présence de palladium et de chlorure.

Le diéthoxy-1,2 butène-3 est mentionné comme co-produit et supposé provenir d'un réarrangement allylique du diéthoxy-1,4-butène-2.

Il a également été proposé (cf. US-A-4,611,082) de dicarbonyler un dialcoxy-1,4-butène-2 dans un solvant polaire, aprotique et non basique, à 80-140°C en présence d'un halogénure d'un métal de transition, le chlorure de palladium s'étant révélé le plus efficace.

Ce même type de réaction a été l'objet d'une étude détaillée au départ du diméthoxy-1,4-butène-2 parue dans "Journal of Molecular Catalysis, 53 (1989) p. 417-432".

Or, les recherches conduites en parallèle par la Demanderesse, en ce qui concerne la préparation des dialcoxybutènes à partir du butadiène-1,3 ont montré que, généralement, on obtient lors de cette préparation un mélange renfermant notamment le dialcoxy-1,4-butène-2 (éther majoritaire) et le dialcoxy-1,2-butène-3, ces deux diéthers étant relativement difficiles à séparer l'un de l'autre.

Il a maintenant été trouvé qu'il est possible de préparer des diesters de l'acide héxène-3-dioïque-1,6 par réaction du monoxyde de carbone sur au moins un dialcoxy-1,2-butène-3 en présence d'un catalyseur à base de palladium.

La présente invention a donc pour objet un procédé de préparation de diesters de l'acide héxène-3-dioïque-1,6 par réaction du monoxyde de carbone sur au moins un dialcoxybutène en présence d'un catalyseur à base de palladium et d'un composé halogéné, en phase liquide, à température élevée et sous une pression supérieure à la pression atmosphérique, caractérisé en ce que le dialcoxybutène est un dialcoxy-1,2-butène-3.

Par dialcoxy-1,2-butène-3, on entend dans le cadre du présent procéde des butènes-3 disubstitués en positions 1 et 2 par des groupes alcoxy identiques ou différents, linéaires, branchés ou cycliques et comportant de 1 à 12 atomes de carbone. De préférence, les deux groupes alcoxy sont identiques et ils comportent avantageusement de 1 à 4 atomes de carbone. Le diméthoxy-1,2-butène-3 et le diethoxy-1,2-butène-3 sont des matières premières particulièrement appropriées, dans le cadre du présent procédé.

Selon une variante avantageuse du procédé selon l'invention, le (ou les) dialcoxy-1,2-butène-3 est (sont) mis en oeuvre sous forme d'un mélange avec le(ou les) dialcoxy-1,4-butène-2, dans lequel ils peuvent représenter une fraction molaire variable, par exemple de 5 à 99 % dudit mélange.

Il a en effet été trouvé de manière surprenante que la sélectivité en diester linéaire recherché (produit dicarbonylé linéaire) au départ de ces diéthers branchés (dialcoxy-1,2-butène-3), qu'ils soient engagés isolément ou en mélange avec les diéthers linéaires correspondants, est suffisamment élevée dans le cadre du présent procédé pour envisager d'utiliser comme matière de départ le mélange du diéther branché et du diéther linéaire avec les proportions dans lesquelles ils se présentent lors de l'étape de leur préparation à partir du butadiène-1,3.

Le procédé selon la présente invention est conduit en présence d'un catalyseur à base de palladium.

Bien que la nature précise de l'espèce (ou des espèces) catalytiquement active(s) dans la réaction en cause ne soit pas totalement élucidée, la Demanderesse suppose que divers composés du palladium et le palladium métallique sont susceptibles d'être des précurseurs utiles dans la mise en oeuvre du présent procédé.

Parmi les sources de palladium pouvant être mises en oeuvre pour réaliser le procédé faisant l'objet de l'invention, on peut citer :

. le palladium métallique déposé le cas échéant sur un support, tel que le charbon, l'alumine, ou la silice,
. $PdCl_2$, $Pd(OAc)_2$
. les sels ou les complexes $\pi$-allyliques de palladium, dont l'anion coordonné au cation Pd est choisi parmi les anions suivants : carboxylates tels que formiate, acétate, propionate, benzoate ; acétylacétonate, halogénures tels que $Cl^-$ et $Br^-$ et de préférence $Cl^-$ ;

On recourt avantageusement au chlorure de palladium.

La quantité précise de catalyseur à mettre en oeuvre, qui peut varier dans de larges limites, dépendra avant tout d'un compromis entre l'efficacité souhaitée et la dépense en catalyseur et des autres conditions choisies pour la réaction. En général de bons résultats peuvent être obtenus avec une concentration en palladium dans le milieu réactionnel, comprise entre $10^{-3}$ et 1 mol/l. Au dessous de $10^{-3}$ mol/l, la cinétique de réaction est fortement ralentie. Des quantités de palladium supérieures à 1 mol/l n'ont d'inconvénients qu'au plan économique. De préférence, cette concentration est comprise entre $10^{-2}$ et 1 mol/l.

Le procédé selon la présente invention est également conduit en présence d'un composé halogéné. La Demanderesse préconise plus particulièrement de recourir à un chlorure ionique dont le cation est choisi dans le groupe constitué par

a) les cations des métaux alcalins,

b) les cations des métaux alcalino-terreux et

c) les cations onium quaternaire d'un élément du groupe VB choisi parmi l'azote et le phosphore, ledit élément étant tétracoordonné à des atomes de carbone, l'azote pouvant être coordonné à deux atomes de phosphore pentavalents.

Pour une bonne mise en oeuvre du procédé selon l'invention, le chlorure d'onium quaternaire présentera un cation onium quaternaire répondant à l'une des formules (I) à (V) ci-après :

I)

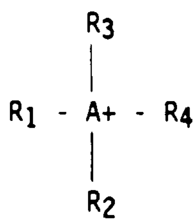

II)

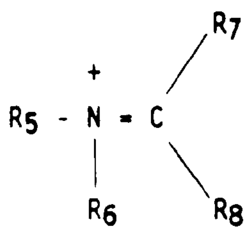

III)

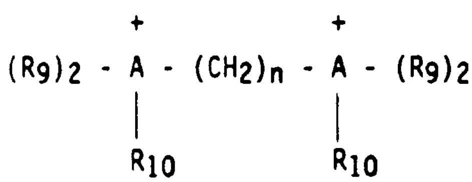

IV)

$$(R_{11})_3 - P = N^+ = P - (R_{11})_3$$

V)

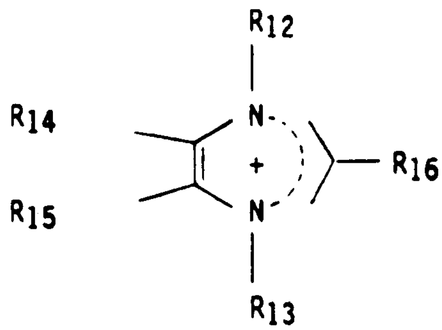

dans lesquelles :

- A représente de l'azote ou du phosphore
- $R_1$, $R_2$, $R_3$, $R_4$ sont identiques ou différents et réprésentent :
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . deux desdits radicaux $R_1$ à $R_4$ pouvant former ensemble un radical alkylène, alcénylène ou alcadiènylène, linéaire ou ramifié et contenant de 3 à 6 atomes de carbone ;
- $R_5$, $R_6$, $R_7$, $R_8$ sont identiques ou différents et représentent :
  . un radical alkyle linéaire ou ramifié contenant de 1 à 4 atomes de carbone ;
  . les radicaux $R_7$ et $R_8$ pouvant former ensemble un radical alkylène, contenant de 3 à 6 atomes de carbone ;
  . les radicaux $R_6$ et $R_7$ ou $R_6$ et $R_8$ pouvant former ensemble un radical alkylène, alcénylène ou alcadiènylène, contenant 4 atomes de carbone et constituant avec N un hétérocycle azoté ;
- $R_9$ représente un radical alkyle linéaire ou ramifié contenant de 1 à 4 atomes de carbone ou un radical phényle ;
- $R_{10}$ représente :
  . un radical alkyle linéaire ou ramifié contenant de 1 à 4 atomes de carbone, semblable ou différent de $R_9$ ;
  . un radical alcényle linéaire ou ramifie, contenant de 2 à 12 atomes de carbone, de préférence de 4 à 8 atomes de carbone ;
- n représente un nombre entier supérieur ou égal à 1 et inférieur ou égal à 10 et de préférence inférieur ou égal à 6 ;
- $R_{11}$ représente un radical aryle contenant de 6 à 10 atomes de carbone, éventuellement substitué par un ou des groupes alkyle contenant de 1 à 4 atomes de carbone, alcoxy, alcoxycarbonyle ou halogéno ;
- $R_{12}$ et $R_{13}$ sont identiques ou différents et ont la signification donnée ci-avant pour $R_1$ à $R_4$ ;
- $R_{14}$ à $R_{16}$ sont identiques ou différents et représentent :
  . un atome d'hydrogène ;
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . les radicaux $R_{14}$ et $R_{15}$ peuvent former ensemble un radical alkylène, alcénylène ou alcadiénylène, linéaire ou ramifié et contenant de 3 à 6 atomes de carbone; ils peuvent ainsi constituer avec les 2 atomes de carbone adjacents du cycle imidazole, un cycle aromatique.

A titre d'exemple de cations onium quaternaire répondant à la formule I on peut citer les cations :

. tétraméthylammonium,
. triéthylméthylammonium

. tributylméthylammonium
. triméthyl(n-propyl)ammonium
. tétraéthylammonium
. tétrabutylammonium
. dodécyltriméthylammonium
. méthyltrioctylammonium
. heptyltributylammonium
. tétrapropylammonium
. tétrapentylammonium
. tétrahexylammonium
. tétraheptylammonium
. tétraoctylammonium
. tétradécylammonium
. butyltripropylammonium
. méthyltributylammonium
. pentyltributylammonium
. méthyldiéthylpropylammonium
. éthyldiméthylpropylammonium
. tétradodécylammonium
. tétraoctadécylammonium
. hexadécyltriméthylammonium
. benzyltriméthylammonium
. benzyldiméthylpropylammonium
. benzyldiméthyloctylammonium
. benzyltributylammonium
. benzyltriéthylammonium
. phényltriméthylammonium
. benzyldiméthyltétradécylammonium
. benzyldiméthylhexadécylammonium
. diméthyldiphénylammonium
. méthyltriphénylammonium
. butène-2 yltriéthylammonium
. N,N-diméthyl-tétraméthylènammonium
. N,N-diéthyl-tétraméthylènammonium
. tétraméthylphosphonium
. tétrabutylphosphonium
. éthyltriméthylphosphonium
. triméthylpentylphosphonium
. octyltriméthylphosphonium
. dodécyltriméthylphosphonium
. triméthylphénylphosphonium
. diéthyldiméthylphosphonium
. dicyclohexyldiméthylphosphonium
. diméthyldiphénylphosponium
. cyclohexyltriméthylphosphonium
. triéthylméthylphosphonium
. méthyl-tri(isopropyl)phosphonium
. méthyl-tri(n-propyl)phosphonium
. méthyl-tri(n-butyl)phosphonium
. méthyl-tri(méthyl-2 propyl)phosphonium
. méthyltricyclohexylphosphonium
. méthyltriphénylphosphonium
. méthyltribenzylphosphonium
. méthyl-tri(méthyl-4 phényl)phosphonium
. méthyltrixylylphosphonium
. diéthylméthylphénylphosphonium
. dibenzylméthylphénylphosphonium
. éthyltriphénylphosphonium

. tétraéthylphosphonium
. éthyl-tri(n-propyl)phosphonium
. triéthylpentylphosphonium
. hexadécyltributylphosphonium
. éthyltriphénylphosphonium
. n-butyl-tri(n-propyl)phosphonium
. butyltriphénylphosphonium
. benzyltriphénylphosphonium
. (β-phényléthyl)diméthylphénylphosphonium
. tétraphénylphosphonium
. triphényl(méthyl-4 phényl)phosphonium
. tétrakis(hydroxyméthyl)phosphonium
. tétrakis(hydroxy-2 éthyl)phosphonium

Parmi les cations répondant à la formule II, on peut citer les cations :

. N-méthylpyridinium
. N-éthylpyridinium
. N-hexadécylpyridinium
. N-méthylpicolinium

Parmi les cations répondant à la formule III, on peut citer les cations :

. bis(triméthylammonium)-1,2 éthane
. bis(triméthylammonium)-1,3 propane
. bis(triméthylammonium)-1,4 butane
. bis(triméthylammonium)-1,3 butane

Parmi les cations répondant à la formule IV, on peut citer les cations :

. bis(triphénylphosphine)iminium
. bis(tritolylphosphine)iminium.

Parmi les cations répondant à la formule V, on peut citer les cations :

. méthyl-1, méthyl-3 imidazolium
. méthyl-1, éthyl-3 imidazolium
. méthyl-1, n-propyl-3 imidazolium
. méthyl-1, n-butyl-3 imidazolium
. méthyl-1, benzyl-3 imidazolium
. méthyl-1, méthyl-2, éthyl-3 benzimidazolium.

On recourt avantageusement à ceux des cations onium répondant à la formule (I) ci-avant dans laquelle :

- A représente du phosphore et,
- $R_1$, $R_2$, $R_3$ et $R_4$ sont identiques ou différents et représentent un radical alkyle linéaire ou ramifié contenant de 1 à 8 atomes de carbone, un radical phényle ou méthyl-4 phényle.

On utilise de préférence un chlorure de tétralkylphosphonium.

Le chlorure de tétrabutylphosphonium disponible et particulièrement efficace est plus spécialement recommandé.

On notera que certains composés du palladium, tel $PBu_4PdCl_3$, peuvent constituer à la fois une source de palladium et un moyen d'introduire au moins une partie du chlorure d'onium quaternaire du sens indiqué ci-avant.

Comme indiqué précédemment, le procédé selon la présente invention peut être mise en oeuvre en utilisant comme chlorure ionique alcalin ou alcalino-terreux. A titre d'exemples de tels chlorures on peut citer LiCl et Ca $Cl_2$ , LiCl se révélant plus particulièrement efficace.

Bien entendu, an peut utiliser dans le cadre de la présente invention un mélange de chlorures minéraux et/ou de chlorures d'onium quaternaire au sens rappelé en tête du présent mémoire.

Il a déjà été proposé dans EP-A-347340 de préparer des diesters de l'acide hexène-3-dioïque-1,6 par réaction du monoxyde de carbone et d'un alcool sur au moins un butène di-substitué par des groupes acyloxy en présence d'un catalyseur à base de palladium et d'un chlorure d'onium quaternaire répondant aux formules I à IV ci-devant.

En général la quantité de chlorure ionique à engager dans le milieu réactionnel sera telle que le rapport molaire $Cl^-/Pd$ soit supérieur ou égal à 1, ce rapport n'ayant de limites supérieures qu'en raison de contraintes économiques et/ou de difficultés de manipulation du milieu réactionnel.

De préférence ledit rapport molaire est compris entre 3 et 100.

Le milieu réactionnel peut, le cas échéant, renfermer un diluant ou solvant organique. Lorsqu'on souhaite opérer en présence d'un diluant ou solvant on recourt à des solvants polaires, aprotiques, basiques ou non

basiques. Parmi les solvants polaires, aprotiques non basiques convenant à la mise en oeuvre de l'invention on mentionnera les nitriles tel l'acétonitrile.

Lorsqu'on choisit de recourir seulement à des chlorures alcalins ou alcalino-terreux, il s'avère utile de conduire la réaction dans un solvant polaire, aprotique et de préférence basique.

Par solvant polaire, aprotique et basique, la Demanderesse entend les composés de formule (VI) :

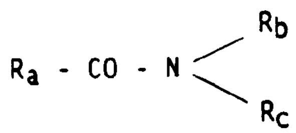

(VI)

dans laquelle $R_a$, $R_b$ et $R_c$, identiques ou différents représentent un radical alkyle, un radical cycloalkyle, un radical aralkyle ou un radical aryle monocyclique ayant au plus 10 atomes de carbone, deux radicaux $R_a$, $R_b$ ou $R_c$, pouvant constituer ensemble un radical unique divalent - $(CH_2)_y$ -, y étant un entier compris entre 3 et 12, $R_a$ pouvant en outre représenter un radical

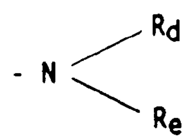

dans lequel $R_c$ et $R_e$, identiques ou différents représentent un radical alkyle ayant au maximum 4 atomes de carbone.

A titre d'exemple de tels solvants, on peut citer : la tétraméthylurée, le N,N-diméthylacétamide, le N,N-diéthylacétamide, le N,N-dicyclohexylacétamide, le N,N-diméthylpropionamide, le N-N-diéthylpropionamide, le N,N-diéthyl-n-butyramide, le N,N-diméthylbenzamide, le N,N-dicyclohexylbenzamide, le N,N-diéthyl-m-toluamide, la N-acétylpyrrolidine, la n-acétylpipéridine, la N-(n-butyryl)pipéridine, la N-méthylpyrrolidone-2, la N-éthylpyrrolidone-2, la N-méthylpipéridone-2, le N-méthyl-epsiloncaprolactame.

La N-méthylpyrrolidone-2 convient particulièrement bien à la mise en oeuvre du présent procédé.

Lorsqu'on recourt à un solvant, sa quantité représente au moins 10 % en volume du milieu réactionnel ; de bons résultats sont obtenus lorsqu'on en utilise de l'ordre de 20 % à 90 % en volume.

La réaction pourra être généralement conduite en phase liquide à une température comprise entre 50 et 180°C, de préférence entre 80 et 150°C, sous une pression d'oxyde de carbone supérieure ou égale à 20 bar et de préférence inférieure ou égale à 250 bar.

Pour une bonne mise en oeuvre de l'invention, la pression d'oxyde de carbone sera de préférence comprise entre 90 et 180 bar.

Des gaz inertes, tels que l'azote, l'argon ou le gaz carbonique, peuvent être présents à côté de l'oxyde de carbone.

En fin de réaction ou du temps imparti à la réaction on récupère le diester recherché par tout moyen approprié, par exemple par extraction et/ou distillation.

Les exemples ci-après illustrent l'invention.

Exemples 1 à 5 ; Essais témoins (a) à (d) :

Dans un autoclave en acier inoxydable (Hastelloy B2) de 20 cm3, préalablement purgé à l'argon, on introduit :

. 8,7 mmol de diméthoxy-1,2-butène-3
. 1,66 mat-g de palladium sous forme de $PdCl_2$
. 6 mmol de chlorure ionique
. le cas échéant, 10 cm3 de solvant.

L'autoclave est fermé hermétiquement, placé dans un four agité et raccordé à l'alimentation de gaz sous pression. On purge le réacteur à froid avec de l'oxyde de carbone et on le porte à 95°C. On régule ensuite la pression à 140 bar. Après réaction, l'autoclave est refroidi et dégazé.

Le mélange réactionnel est alors analysé par chromatographie en phase gazeuse.

Les conditions particulières ainsi que les résultats obtenus figurent au tableau I ci-après dans lequel t représente la durée de réaction en température, HD (%) représente la quantité molaire d'hexène-3-dioate de

méthyle formée pour 100 moles de diméthoxy-1,2-butène-3 chargées, et TT (%) représente le taux de transformation du diméthoxy-1,2-butène-3.

DMI Cl = chlorure de N,N-diméthylimidazolium
NMp = N-méthylpyrrolidone-2
p-DCB = paradichlorobenzène

TABLEAU I

| Réf | Chlorure ionique | Solvant | t (h) | TT (%) | H3D (%) |
|---|---|---|---|---|---|
| 1 | $PBu_4Cl$ | néant | 6 | 100 | 80 |
| 2 | " | NMP | " | " | 92,5 |
| 3 | LiCl | NMP | " | " | 86 |
| 4 | $NMe_4Cl$ | NMP | " | " | 82 |
| 5 | DMICl | NMP | " | " | 91 |
| a | néant | éthanol | " | " | 3 |
| b | " | acétonitrile | " | 37 | 8 |
| c | " | NMP | " | 57 | 9 |
| d | " | p-DCB | " | 100 | 38 |

Exemples 6 et 7 :

Dans un autoclave en acier inoxydable (Hastelloy B2) de 125 cm3, préalablement purgé à l'argon, on introduit :

- 25 mmol de diméthoxy-1,2 butène-3
- 5 mmol de palladium sous forme de $PdCl_2$
- du chlorure de tétrabutylphosphonium et,
- le cas échéant, 30 cm3 de solvant.

L'autoclave est alors fermé et on procède comme décrit pour les exemples 1 à 5 (T = 95°C ; P = 140 bar).

Les conditions particulières ainsi que les résultats obtenus sont rassemblés dans le tableau II ci-après :

TABLEAU II

| Réf | $PBu_4Cl$ mmol | Solvant | t (h) | TT (%) | H3D (%) |
|---|---|---|---|---|---|
| 6 | 5 | acétonitrile | 8 | 96 | 72 |
| 7 | 27 | néant | 8 | 99 | 80 |

Exemples 8 et 9 :

Dans l'autoclave et selon le mode opératoire décrits ci-avant, on reproduit l'exemple 7 sur une charge modifiée comme suit :

- on introduit un mélange de diméthoxy-1,4-butène-2 (DMB-1,4) et de diméthoxy-1,2-butène-3 (DMB-1,2)
- on introduit 19 mmol de $PBu_4Cl$.

Les conditions particulières ainsi que les résultats obtenus sont rassemblés dans le tableau III ci-après :

TABLEAU III

| Réf | DMB-1,4 mmol | DMB-1,2 mmol | t (h) | H3D (%) |
|---|---|---|---|---|
| 8 | 18,5 | 8 | 4,8 | 65 |
| 9 | 8 | 17,5 | 5,7 | 74 |

Dans ces 2 essais, la transformation des diméthoxybutènes est totale.

## Revendications

1. Procédé de préparation de diesters de l'acide hexène-3-dioïque-1,6 par réaction du monoxyde de carbone sur au moins un dialcoxybutène en présence d'un catalyseur à base de palladium et d'un composé halogéné, en phase liquide, à température élevée et sous une pression supérieure à la pression atmosphérique, caractérisé en ce que le dialcoxybutène est un dialcoxy-1,2-butène-3.

2. Procédé selon la revendication 1, caractérisé en ce que l'on engage à la réaction un mélange de dialcoxy-1,2 butène-3 et de dialcoxy-1,4-butène-2.

3. Procédé selon la revendication 1 ou 2, caractérisé en ce que le composé halogéné est un chlorure ionique dont le cation est choisi dans le groupe constitué par
   a) les cations des métaux alcalins,
   b) les cations des métaux alcalino-terreux et
   c) les cations onium quaternaire d'un élément du groupe VB choisi parmi l'azote et le phosphore, ledit élément étant tétracoordonné à des atomes de carbone, l'azote pouvant être coordonné à deux atomes de phosphore pentavalents.

4. Procédé selon l'une quelconque des revendications précédentes, caractérisé en ce que le composé halogéné est un chlorure d'onium quaternaire d'un élément du groupe VB choisi parmi l'azote et le phosphore, ledit élément étant tétracoordonné à des atomes de carbone, l'azote pouvant être coordonné à deux atomes de phosphore pentavalents.

5. Procédé selon la revendication 1 ou 2, caractérisé en ce que le cation onium quaternaire répond à l'une des formules (I) à (V) suivantes :
   I)

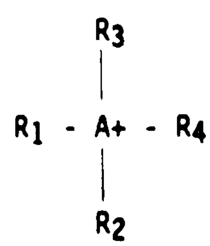

II)

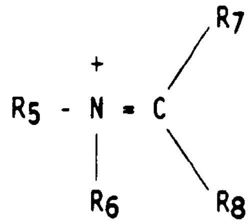

III)

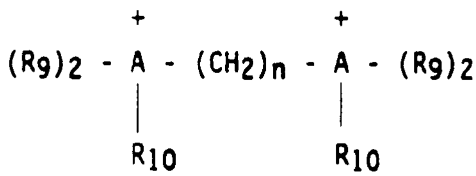

IV)

$$(R_{11})_3 - P = N^+ = P - (R_{11})_3$$

V)

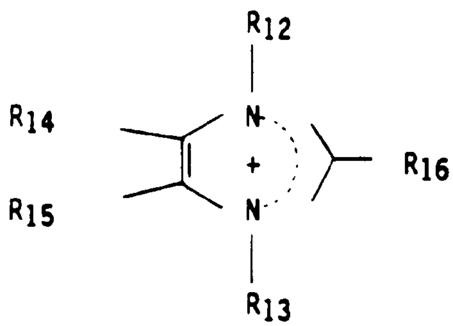

dans lesquelles :

- A représente de l'azote ou du phosphore
- $R_1$, $R_2$,$R_3$, $R_4$ sont identiques ou différents et réprésentent :
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . deux desdits radicaux $R_1$ à $R_4$ pouvant former ensemble un radical alkylène, alcénylène ou alcadiènylène, linéaire ou ramifié et contenant de 3 à 6 atomes de carbone ;
- $R_5$, $R_6$, $R_7$, $R_8$ sont identiques ou différents et représentent :
  . un radical alkyle linéaire ou ramifié contenant de 1 à 4 atomes de carbone ;
  . les radicaux $R_7$ et $R_8$ pouvant former ensemble un radical alkylène, contenant de 3 à 6 atomes de carbone ;
  . les radicaux $R_6$ et $R_7$ ou $R_6$ et $R_8$ pouvant former ensemble un radical alkylène, alcénylène ou alcadiènylène, contenant 4 atomes de carbone et constituant avec N un hétérocycle azoté ;
- $R_9$ représente un radical alkyle linéaire ou ramifié contenant de 1 à 4 atomes de carbone ou un radical phényle ;
- $R_{10}$ représente :
  . un radical alkyle linéaire ou ramifié contenant de 1 à 4 atomes de carbone, semblable ou dif-

férent de $R_9$ ;
. un radical alcényle linéaire ou ramifié, contenant de 2 à 12 atomes de carbone, de préférence de 4 à 8 atomes de carbone ;

- n représente un nombre entier supérieur ou égal à 1 et inférieur ou égal à 10 et de préférence inférieur ou égal à 6 ;
- $R_{11}$ représente un radical aryle contenant de 6 à 10 atomes de carbone, éventuellement substitué par un ou des groupes alkyle contenant de 1 à 4 atomes de carbone, alcoxy, alcoxycarbonyle ou halogéno ;
- $R_{12}$ et $R_{13}$ sont identiques ou différents et ont la signification donnée ci-avant pour $R_1$ à $R_4$ ;
- $R_{14}$ à $R_{16}$ sont identiques ou différents et représentent :
  . un atome d'hydrogène ;
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . les radicaux $R_{14}$ et $R_{15}$ peuvent former ensemble un radical alkylène, alcénylène ou alcadiénylène, linéaire ou ramifié et contenant de 3 à 6 atomes de carbone, ils peuvent ainsi constituer avec les 2 atomes de carbone adjacents du cycle imidazole, un cycle aromatique.

**6.** Procédé selon la revendication 5, caractérisé en ce que le cation d'onium quaternaire répond à la formule (I) dans laquelle :
- A représente du phosphore et,
- $R_1$, $R_2$, $R_3$ et $R_4$ sont identiques ou différents et représentent un radical alkyle linéaire ou ramifié contenant de 1 à 8 atomes de carbone, un radical phényle ou méthyl-4 phényle.

**7.** Procédé selon l'une quelconque des revendications précédentes, caractérisé en ce que le composé halogéné est le chlorure de tétrabutylphosphonium.

**8.** Procédé selon l'une quelconque des revendications 1 à 3, caractérisé en ce que le composé halogéné est un chlorure alcalin ou alcalino terreux.

**9.** Procédé selon l'une quelconque des revendications 1 à 3, caractérisé en ce que le composé halogéné est le chlorure de lithium.

**10.** Procédé selon l'une quelconque des revendications 8 à 9, caractérisé en ce que la réaction est conduite dans un solvant polaire, aprotique et basique.

**11.** Procédé selon la revendication 10, caractérisé en ce que le solvant est choisi parmi les composés de formule (VI) :

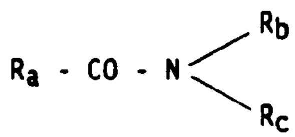

(VI)

dans laquelle $R_a$, $R_b$ et $R_c$, identiques ou différents représentent un radical alkyle, un radical cycloalkyle, un radical aralkyle ou un radical aryle monocyclique ayant au plus 10 atomes de carbone, deux radicaux $R_a$, $R_b$ ou $R_c$, pouvant constituer ensemble un radical unique divalent $-(CH_2)y-$, y étant un entier compris entre 3 et 12, $R_a$ pouvant en outre représenter un radical

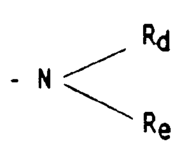

dans lequel $R_d$ et $R_e$, identiques ou différents représentent un radical alkyle ayant au maximum 4 atomes de carbone.

12. Procédé selon la revendication 10 ou 11 caractérisé en ce que la quantité de solvant représente au moins 10 % en volume du milieu réactionnel.

13. Procédé selon l'une quelconque des revendications 10 à 12 caractérisé en ce que le solvant est la N-méthylpyrrolidone-2.

14. Procédé selon l'une quelconque des revendications précédentes, caractérisé en ce que le rapport molaire de l'anion chlorure au palladium est compris entre 3 et 100.

15. Procédé selon l'une quelconque des revendications précédentes, caractérisé en ce que la concentration en palladium dans le milieu réactionnel est comprise entre $10^{-3}$ et 1 mol/l.

16. Procédé selon l'une quelconque des revendications précédentes, caractérisé en ce que la température de réaction est comprise entre 50 et 180°C et, de préférence, entre 80 et 150°C.

17. Procédé selon l'une quelconque des revendications précédentes, caractérisé en ce que la pression est supérieure ou égale à 20 bar et de préférence, inférieure ou égale à 250 bar.

18. Procédé selon l'une quelconque des revendications précédentes, caractérisé en ce que la pression est comprise entre 90 et 180 bar.

19. Procédé selon l'une quelconque des revendications précédentes, caractérisé en ce que le palladium est engagé sous forme de chlorure de palladium.

## Claims

1. Process for the preparation of 3-hexene-1,6-dioic acid diesters by reaction of carbon monoxide with at least one dialkoxybutene in the presence of a palladium-based catalyst and a halogenated compound, in the liquid phase, at high temperature and under a pressure greater than atmospheric pressure, characterized in that the dialkoxybutene is a 1,2-dialkoxy-3-butene.

2. Process according to claim 1, characterized in that a mixture of 1,2-dialkoxy-3-butene and 1,4-dialkoxy-2-butene is employed in the reaction.

3. Process according to claim 1 or 2, characterized in that the halogenated compound is an ionic chloride whose cation is chosen from the group consisting of
   a) alkali metal cations,
   b) alkaline-earth metal cations, and
   c) quaternary onium cations of a group VB element chosen from nitrogen and phosphorus, the said element being tetracoordinated to carbon atoms, the nitrogen being able to be coordinated to two pentavalent phosphorus atoms.

4. Process according to any one of the preceding claims, characterized in that the halogenated compound is a quaternary onium chloride of a group VB element chosen from nitrogen and phosphorus, the said element being tetracoordinated to carbon atoms, the nitrogen being able to be coordinated to two pentavalent phosphorus atoms.

5. Process according to claim 1 or 2, characterized in that the quaternary onium cation corresponds to one of the following formulae (I) to (V) :

I)

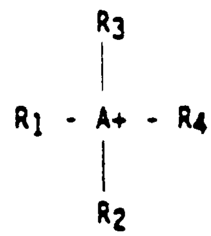

II)

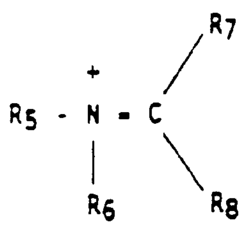

III)

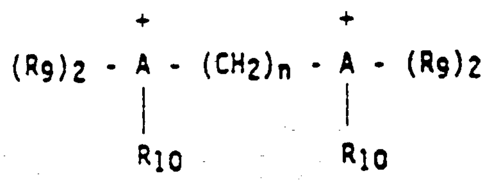

IV)

$$(R_{11})_3 - P = N^+ = P - (R_{11})_3$$

V)

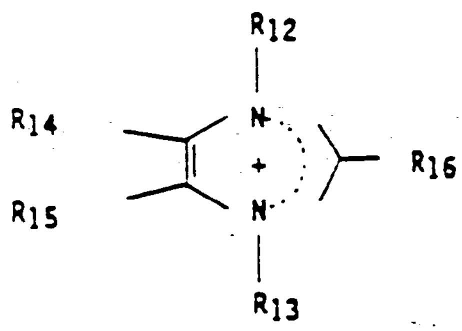

in which formulae:

- A represents nitrogen or phosphorus
- $R_1$, $R_2$, $R_3$ and $R_4$ are identical or different and represent:
  - a linear or branched alkyl radical containing 1 to 16 carbon atoms, optionally substituted by a phenyl, hydroxyl, halo, nitro, alkoxy or alkoxycarbonyl group;
  - a linear or branched alkenyl radical containing 2 to 12 carbon atoms, preferably 4 to 8 carbon atoms,
  - an aryl radical containing 6 to 10 carbon atoms, optionally substituted by one or more alkyl radical(s) containing 1 to 4 carbon atoms, or alkoxy, alkoxycarbonyl or halo radicals;
  - two of the said radicals $R_1$ to $R_4$ being able jointly to form a linear or branched alkylene, alkenylene or alkadienylene radical containing 3 to 6 carbon atoms;

- $R_5$, $R_6$, $R_7$ and $R_8$ are identical or different and represent:
    - a linear or branched alkyl radical containing 1 to 4 carbon atoms;
    - the radicals $R_7$ and $R_8$ being able jointly to form an alkylene radical containing 3 to 6 carbon atoms;
    - the radicals $R_6$ and $R_7$ or $R_8$ and $R_8$ being able jointly to form an alkylene, alkenylene or an alkadienylene radical containing 4 carbon atoms and constituting with N a nitrogenous heterocycle;
- $R_9$ represents a linear or branched alkyl radical containing 1 to 4 carbon atoms, or a phenyl radical;
- $R_{10}$ represents:
    - a linear or branched alkyl radical containing 1 to 4 carbon atoms, similar to or different from $R_9$;
    - a linear or branched alkenyl radical containing 2 to 12 carbon atoms, preferably 4 to 8 carbon atoms;
- n represents an integer greater than or equal to 1 and less than or equal to 10, and preferably less than or equal to 6;
- $R_{11}$ represents an aryl radical containing 6 to 10 carbon atoms, optionally substituted by one or more alkyl group(s) containing 1 to 4 carbon atoms, or alkoxy, alkoxycarbonyl or halo groups;
- $R_{12}$ and $R_{13}$ are identical or different and have the meaning given earlier for $R_1$ to $R_4$;
- $R_{14}$ to $R_{16}$ are identical or different and represent:
    - a hydrogen atom
    - a linear or branched alkyl radical containing 1 to 16 carbon atoms, optionally substituted by a phenyl, hydroxyl, halo, nitro, alkoxy, or alkoxycarbonyl group;
    - a linear or branched alkenyl radical containing 2 to 12 carbon atoms, preferably 4 to 8 carbon atoms,
    - an aryl radical containing 6 to 10 carbon atoms, optionally substituted by one or more alkyl radical(s) containing 1 to 4 carbon atoms, or alkoxy, alkoxycarbonyl or halogeno radicals;
    - the radicals $R_{14}$ and $R_{15}$ may jointly form a linear or branched alkylene, alkenylene or alkadienylene radical containing 3 to 6 carbon atoms; they can thus constitute, with the 2 adjacent carbon atoms of the imidazole ring, an aromatic ring.

**6.** Process according to claim 5, characterized in that the quaternary onium cation corresponds to the formula (I) in which:
- A represents phosphorus, and
- $R_1$, $R_2$, $R_3$ and $R_4$ are identical or different and represent a linear or branched alkyl radical containing 1 to 8 carbon atoms, a phenyl radical or a 4-methylphenyl radical.

**7.** Process according to any of the preceding claims, characterized in that the halogenated compound is tetrabutylphosphonium chloride.

**8.** Process according to any one of claims 1 to 3, characterized in that the halogenated compound is an alkali metal chloride or an alkaline-earth metal chloride.

**9.** Process according to any one of claims 1 to 3, characterized in that the halogenated compound is lithium chloride.

**10.** Process according to either of claims 8 and 9, characterized in that the reaction is performed in an aprotic and basic polar solvent.

**11.** Process according to claim 10, characterized in that the solvent is chosen from the compounds of formula (VI):

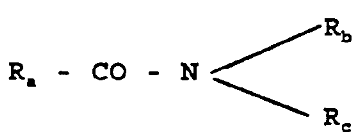

(VI)

in which $R_a$, $R_b$ and $R_c$, which may be identical or different, represent an alkyl radical, a cycloalkyl radical, an aralkyl radical or a monocyclic aryl radical having not more than 10 carbon atoms, two radicals $R_a$, $R_b$

or $R_c$ being able jointly to constitute a single divalent radical - $(CH_2)_y$ -, y being an integer between 3 and 12, and $R_a$ being able, in addition, to represent a radical

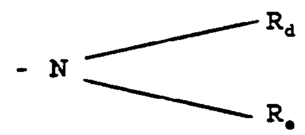

in which $R_c$ and $R_a$, which may be identical or different, represent an alkyl radical having at most 4 carbon atoms.

**12.** Process according to claim 10 or 11, characterized in that the quantity of solvent represents at least 10% by volume of the reaction mixture.

**13.** Process according to any one of claims 10 to 12, characterized in that the solvent is N-methyl-2-pyrrolidone.

**14.** Process according to any one of the preceding claims, characterized in that the molar ratio of the chloride anion to the palladium is between 3 and 100.

**15.** Process according to any one of the preceding claims, characterized in that the palladium concentration in the reaction mixture is between $10^{-3}$ and 1 mol/l.

**16.** Process according to any one of the preceding claims, characterized in that the reaction temperature is between 50 and 180°C, and preferably between 80 and 150°C.

**17.** Process according to any one of the preceding claims, characterized in that the pressure is greater than or equal to 20 bar, and preferably less than or equal to 250 bar.

**18.** Process according to any one of the preceding claims, characterized in that the pressure is between 90 and 180 bar.

**19.** Process according to any one of the preceding claims, characterized in that the palladium is employed in the form of palladium chloride.

**Patentansprüche**

**1.** Verfahren zur Herstellung von Diestern der 3-Hexen-1,6-disäure durch Umsetzung von Kohlenmonoxid mit mindestens einem Dialkoxybuten in Gegenwart eines Katalysators auf Palladium-Basis und einer Halogenverbindung in flüssiger Phase bei erhöhter Temperatur und unter höherem Druck als Atmosphärendruck, dadurch gekennzeichnet, daß das Dialkoxybuten ein 1,2-Dialkoxy-3-buten ist.

**2.** Verfahren nach Anspruch 1, dadurch gekennzeichnet, daß man für die Reaktion eine Mischung von 1,2-Dialkoxy-3-buten und 1,4-Dialkoxy-2-buten einsetzt.

**3.** Verfahren nach Anspruch 1 oder 2, dadurch gekennzeichnet, daß die Halogenverbindung ein ionisches Chlorid ist, dessen Kation ausgewählt ist aus der Gruppe, die besteht aus

a) den Kationen der Alkalimetalle,

b) den Kationen der Erdalkalimetalle und

c) den quaternären Onium-Kationen eines Elements der Gruppe VB, ausgewählt aus Stickstoff und Phosphor, wobei besagtes Element vierfach mit Kohlenstoffatomen koordiniert ist, wobei der Stickstoff mit zwei fünfwertigen Phosphoratomen koordiniert sein kann.

**4.** Verfahren nach irgendeinem der vorangehenden Ansprüche, dadurch gekennzeichnet, daß die Halogenverbindung ein quaternäres Oniumchlorid eines Elements der Gruppe VB ist, ausgewählt aus Stickstoff und Phosphor, wobei das Element vierfach mit Kohlenstoffatomen koordiniert ist, wobei der Stickstoff mit zwei fünfwertigen Phosphoratomen koordiniert sein kann.

5. Verfahren nach Anspruch 1 oder 2, dadurch gekennzeichnet, daß das quaternäre Onium-Kation einer der folgenden Formeln (I) bis (V) entspricht:

I)

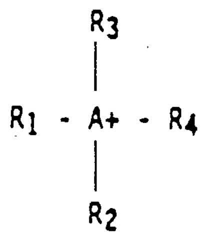

II)

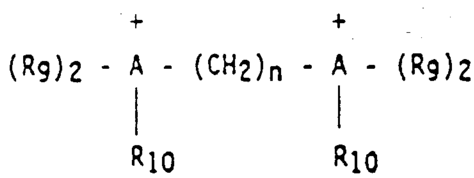

III)

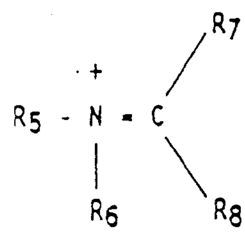

IV)

$$(R_{11})_3 - P = N^+ = P - (R_{11})_3$$

V)

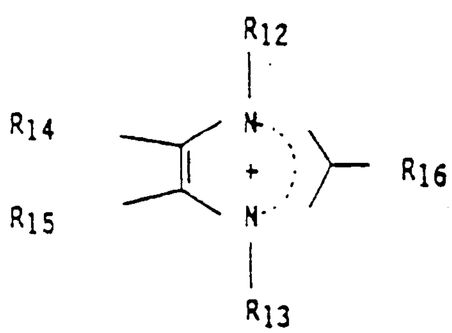

in denen:

- A Stickstoff oder Phosphor darstellt
- $R_1$, $R_2$, $R_3$, $R_4$ identisch oder verschieden sind und darstellen:
  . eine linearen oder verzweigten Alkylrest mit 1 bis 16 Kohlenstoffatomen, gegebenenfalls durch eine Phenyl-, Hydroxy-, Halogen-, Nitro-, Alkoxy- oder Alkoxycarbonylgruppe substituiert;
  . einen linearen oder verzweigten Alkenylrest mit 2 bis 12 Kohlenstoffatomen, vorzugsweise 4 bis 8 Kohlenstoffatomen,
  . einen Arylrest mit 6 bis 10 Kohlenstoffatomen, gegebenenfalls durch (einen) Alkylrest(e) mit

1 bis 4 Kohlenstoffatomen, Alkoxy-, Alkoxycarbonyl- oder Halogenrest(e) substituiert;
. wobei zwei der Reste $R_1$ bis $R_4$ zusammen einen Alkylen-, Alkenylen- oder Alkadienylenrest, linear oder verzweigt und 3 bis 6 Kohlenstoffatome enthaltend, bilden können;

- $R_5$, $R_6$, $R_7$, $R_8$ identisch oder verschieden sind und darstellen:
  . einen linearen oder verzweigten Alkylrest mit 1 bis 4 Kohlenstoffatomen;
  . wobei die Reste $R_7$ und $R_8$ zusammen einen Alkylenrest mit 3 bis 6 Kohlenstoffatomen bilden können;
  . wobei die Reste $R_6$ und $R_7$ oder $R_8$ und $R_8$ zusammen einen Alkylen-, Alkenylen- oder Alkadienylenrest, der 4 Kohlenstoffatome enthält und mit N einen Stickstoffheterocyclus aufbaut, bilden können;
- $R_9$ einen linearen oder verzweigten Alkylrest mit 1 bis 4 Kohlenstoffatomen oder einen Phenylrest darstellt;
- $R_{10}$ darstellt:
  . einen linearen oder verzweigten Alkylrest mit 1 bis 4 Kohlenstoffatomen, gleich wie oder verschieden von $R_9$;
  . einen linearen oder verzweigten Alkenylrest mit 2 bis 12 Kohlenstoffatomen, vorzugsweise 4 bis 8 Kohlenstoffatomen;
- n eine ganze Zahl oberhalb von oder gleich 1 und unterhalb von oder gleich 10 und vorzugsweise unterhalb von oder gleich 6 darstellt;
- $R_{11}$ einen Arylrest mit 6 bis 10 Kohlenstoffatomen, gegebenenfalls durch (eine) Alkylgruppe(n) mit 1 bis 4 Kohlenstoffatomen, Alkoxy-, Alkoxycarbonyl- oder Halogengruppe(n) substituiert, darstellt;
- $R_{12}$ und $R_{13}$ identisch oder verschieden sind und die vorstehend angegebene Bedeutung für $R_1$ bis $R_4$ aufweisen;
- $R_{14}$ bis $R_{16}$ identisch oder verschieden sind und darstellen:
  . ein Wasserstoffatom;
  . einen linearen oder verzweigten Alkylrest mit 1 bis 16 Kohlenstoffatomen, gegebenenfalls durch eine Phenyl-, Hydroxy-, Halogen-, Nitro-, Alkoxy- oder Alkoxycarbonylgruppe substituiert;
  . einen linearen oder verzweigten Alkenylrest mit 2 bis 12 Kohlenstoffatomen, vorzugsweise 4 bis 8 Kohlenstoffatomen,
  . einen Arylrest mit 6 bis 10 Kohlenstoffatomen, gegebenenfalls durch (einen) Alkylrest(e) mit 1 bis 4 Kohlenstoffatomen, Alkoxy-, Alkoxycarbonyl- oder Halogenrest(e) substituiert;
  . wobei die Reste $R_{14}$ und $R_{15}$ zusammen einen Alkylen-, Alkenylen- oder Alkadienylenrest, linear oder verzweigt und 3 bis 6 Kohlenstoffatome enthaltend, bilden können; sie können so mit den zwei benachbarten Kohlenstoffatomen des Imidazolrings einen aromatischen Ring bilden.

**6.** Verfahren nach Anspruch 5, dadurch gekennzeichnet, daß das quaternäre Onium-Kation der Formel (I) entspricht, in der:
- A Phosphor darstellt und
- $R_1$, $R_2$, $R_3$ und $R_4$ identisch oder verschieden sind und einen linearen oder verzweigten Alkylrest mit 1 bis 8 Kohlenstoffatomen, einen Phenyl- oder 4-Methylphenylrest darstellen.

**7.** Verfahren nach irgendeinem der vorangehenden Ansprüche, dadurch gekennzeichnet, daß die Halogenverbindung Tetrabutylphosphoniumchlorid ist.

**8.** Verfahren nach Irgendeinem der Ansprüche 1 bis 3, dadurch gekennzeichnet, daß die Halogenverbindung ein Alkalioder Erdalkalichlorid ist.

**9.** Verfahren nach irgendeinem der Ansprüche 1 bis 3, dadurch gekennzeichnet, daß die Halogenverbindung Lithiumchlorid ist.

**10.** Verfahren nach irgendeinem der Ansprüche 8 bis 9, dadurch gekennzeichnet, daß die Reaktion in einem polaren, aprotischen und basischen Lösungsmittel durchgeführt wird.

**11.** Verfahren nach Anspruch 10, dadurch gekennzeichnet, daß das Lösungsmittel ausgewählt ist aus den Verbindungen der Formel (VI):

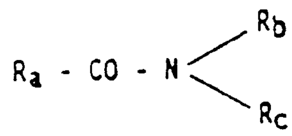 (VI)

in der $R_a$, $R_b$ und $R_c$, identisch oder verschieden, einen Alkylrest, einen Cycloalkylrest, einen Aralkylrest oder einen monocyclischen Arylrest mit nicht mehr als 10 Kohlenstoffatomen darstellen, wobei zwei Reste $R_a$, $R_b$ oder $R_c$ zusammen einen einzigen zweiwertigen Rest - $(CH_2)_y$ - aufbauen können, wobei y eine ganze Zahl eingeschlossen zwischen 3 und 12 ist, wobei $R_a$ darüberhinaus einen Rest

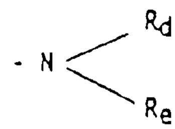

darstellen kann, in dem $R_d$ und $R_e$, identisch oder verschieden, einen Alkylrest mit maximal 4 Kohlenstoffatomen darstellen.

**12.** Verfahren nach Anspruch 10 oder 11, dadurch gekennzeichnet, daß die Lösungsmittelmenge mindestens 10 Volumen-% des Reaktionsmediums ausmacht.

**13.** Verfahren nach irgendeinem der Ansprüche 10 bis 12, dadurch gekennzeichnet, daß das Lösungsmittel N-Methylpyrrolidon-2 ist.

**14.** Verfahren nach irgendeinem der vorangehenden Ansprüche, dadurch gekennzeichnet, daß das Molverhältnis des Chloridanions zu Palladium zwischen 3 und 100 eingeschlossen ist.

**15.** Verfahren nach irgendeinem der vorangehenden Ansprüche, dadurch gekennzeichnet, daß die Palladiumkonzentration im Reaktionsmedium zwischen $10^{-3}$ und 1 Mol/l eingeschlossen ist.

**16.** Verfahren nach irgendeinem der vorangehenden Ansprüche, dadurch gekennzeichnet, daß die Reaktionstemperatur zwischen 50 und 180°C und vorzugsweise zwischen 80 und 150°C eingeschlossen ist.

**17.** Verfahren nach irgendeinem der vorangehenden Ansprüche, dadurch gekennzeichnet, daß der Druck oberhalb von oder gleich 20 bar und vorzugsweise unterhalb von oder gleich 250 bar ist.

**18.** Verfahren nach irgendeinem der vorangehenden Ansprüche, dadurch gekennzeichnet, daß der Druck zwischen 90 und 180 bar eingeschlossen ist.

**19.** Verfahren nach irgendeinem der vorangehenden Ansprüche, dadurch gekennzeichnet, daß das Palladium in Form von Palladiumchlorid eingesetzt wird.